# EUROPEAN PATENT APPLICATION

(11) **EP 1 815 830 A1**
(43) Date of publication of application: **08.08.2007**
(21) Application number: 07006435.7
(22) Date of filing: 05.11.2003
(51) Int. Cl.: A61F 7/00, A47K 13/00

(54) **Vacuum assisted closure system with provision for introduction of agent**

(30) Priority: 08.11.2002 US 290938
(62) Divisional of application: 03811249.6
(71) Applicant: KCI Licensing, Inc., San Antonio, TX 78265-9508 (US)
(72) Inventor: Beard, Mark, Ferndown Dorset, BH22 0HE (GB); Whyte, David, Wareham Dorset, BH20 4JL (GB); Stacy, Peter, Ferndown Dorset, BH22 9QE (GB); Coward, Chris, Wareham Dorset, HB20 4SF (GB)
(74) Representative: Lockey, Robert Alexander

(57) **Abstract**

An apparatus for cooling a patient to a temperature below normal body temperature, the apparatus comprising a patient-enclosing air tent having an inlet and outlet connected to an air-cooling system, the air-cooling system recirculating air through the air tent to conserve energy, a plurality of air-bags collectively forming a patient supporting surface, a high air pressure source in fluid communication with the air-bags, and a fluid connection between the air-bags and the air cooling system wherein the air-bags can be pressurised with either relatively low pressure cold air which assists in cooling the patient but provides relatively little support or with relatively high pressure air which is sufficient to support the patient but which provides relatively less cooling effect.

## Description

### RELATED APPLICATION:

This application claims priority to United States provisional patent application Serial No. 60/127,595 entitled VACUUM ASSISTED CLOSURE SYSTEM WITH PROVISION FOR INTRODUCTION OF AGENT filed April 2, 1999. By this reference, the full disclosure, including the drawings, of U.S. provisional patent application Serial No. 60/127,595 is incorporated herein.

### TECHNICAL FIELD:

The present invention relates to the healing of wounds. More specifically, the present invention relates to the vacuum assisted closure (VAC) of wounds wherein a growth factor or other agent is introduced to a wound site through grafting with a VAC pad in order to facilitate wound healing.

### BACKGROUND ART:

Wound closure involves the inward migration of epithelial and subcutaneous tissue adjacent the wound. This migration is ordinarily assisted through the inflammatory process, whereby blood flow is increased and various functional cell types are activated. Through the inflammatory process, blood flow through damaged or broken vessels is stopped by capillary level occlusion, whereafter cleanup and rebuilding operations may begin. Unfortunately, this process is hampered when a wound is large or has become infected. In such wounds, a zone of stasis (i.e. an area in which localized swelling of tissue restricts the flow of blood to the tissues) forms near the surface of the wound.

Without sufficient blood flow, the epithelial and subcutaneous tissues surrounding the wound not only receive diminished oxygen and nutrients, but are also less able to successfully fight bacterial infection and thus are less able to naturally close the wound. Until recently, such difficult wounds were addressed only through the use of sutures or staples. Although still widely practiced and often effective, such mechanical closure techniques suffer a major disadvantage in that they produce tension on the skin tissue adjacent the wound. In particular, the tensile force required in order to achieve closure using sutures or staples causes very high localized stresses at the suture or staple insertion point. These stresses commonly result in the rupture of the tissue at the insertion points, which can eventually cause wound dehiscence and additional tissue loss.

Additionally, some wounds harden and inflame to such a degree due to infection that closure by stapling or suturing is not feasible. Wounds not reparable by suturing or stapling generally require prolonged hospitalization, with its attendant high cost, and major surgical procedures, such as grafts of surrounding tissues. Examples of wounds not readily treatable with staples or suturing include large, deep, open wounds; decubitus ulcers; ulcers resulting from chronic osteomyelitis; and partial thickness bums that subsequently develop into full thickness bums.

As a result of these and other shortcomings of mechanical closure devices, methods and apparatus for draining wounds by applying continuous negative pressures have been developed. When applied over a sufficient area of the wound, such negative pressures have been found to promote the migration toward the wound of epithelial and subcutaneous tissues. In practice, the application to a wound of negative pressure, commonly referred to as vacuum assisted closure (VAC) therapy, typically involves mechanical-like contraction of the wound with simultaneous removal of excess fluid. In this manner, VAC therapy augments the body's natural inflammatory process while alleviating many of the known intrinsic side effects, such as the production of edema caused by increased blood flow absent the necessary vascular structure for proper venous return.

While VAC therapy has been highly successful in the promotion of wound closure, healing many wounds previously thought largely untreatable, some difficulty remains. Because the inflammatory process is very unique to the individual patient, even the addition of VAC therapy does not result in a fast enough response, especially during the occlusion and initial cleanup and rebuilding stages, for adequate healing of some wounds. It is therefore a principle object of the present invention to provide a method and apparatus whereby the known VAC therapy modalities are improved through the introduction of growth factors and/or other agents that facilitate wound healing.

### DISCLOSURE OF THE INVENTION:

In accordance with the foregoing objects, the present invention - a method and apparatus for the introduction to a wound undergoing vacuum assisted closure (VAC) therapy of a wound healing agent - generally comprises a foam pad for insertion substantially into a wound site and a wound drape for sealing enclosure of the foam pad at the wound site. According to the invention, the foam pad is placed in fluid communication with a vacuum source for promotion of fluid drainage. Additionally, the foam pad is predisposed, through grafting or other techniques known to those of ordinary skill in the art, with basic fibroblast growth factor (bFGF), anti-microbials or other factors, also known to those of ordinary skill in the art, for the promotion of increased wound healing.

According to the preferred method of the present invention, a growth factor or other wound healing agent is added to the previously known VAC therapy through modification as necessary of the VAC system's foam pad. Such growth factors as the basic fibroblast growth factor (bFGF) are known to accelerate wound healing due to their potent angiogenesis and granulation tissue formation activities. As has been demonstrated even with difficult to heal wounds, such as infected wounds, burn wounds and diabetic wounds, the resultant activities lead to the rapid reepitherialization and contraction of the wound. The combination of VAC therapy with growth factor introduction, through the modification of the foam pad and predisposition thereof with the bFGF, is therefore thought to be an important contribution to the wound healing arts.

Finally, many other features, objects and advantages of the present invention will be apparent to those of ordinary skill in the relevant arts, especially in light of the foregoing discussions, the following drawing and exemplary detailed description and the claims appended hereto.

### BRIEF DESCRIPTION OF THE DRAWINGS:

Although the scope of the present invention is much broader than any particular embodiment, a detailed description of the preferred embodiment follows together with an illustrative figure, wherein like reference numerals refer to like components, and wherein:

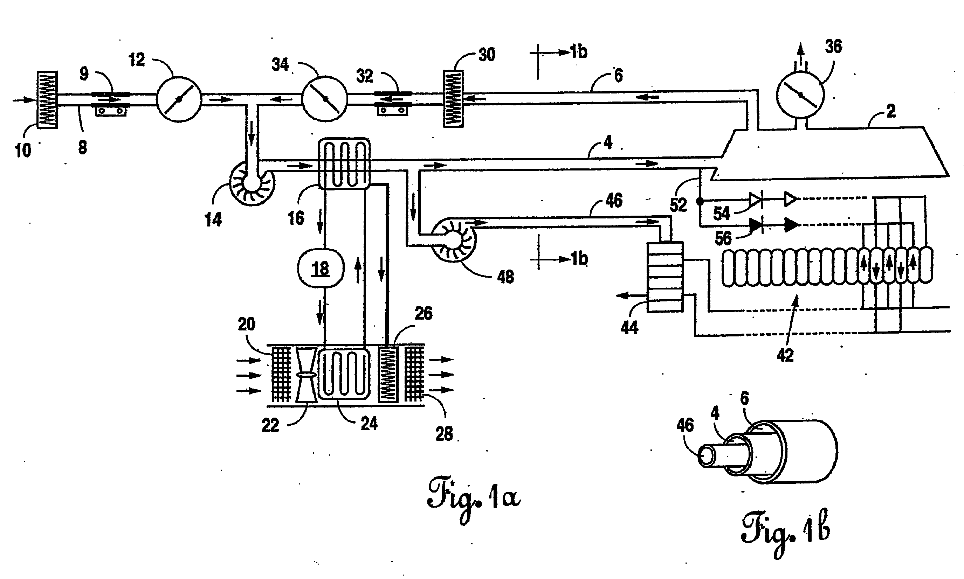

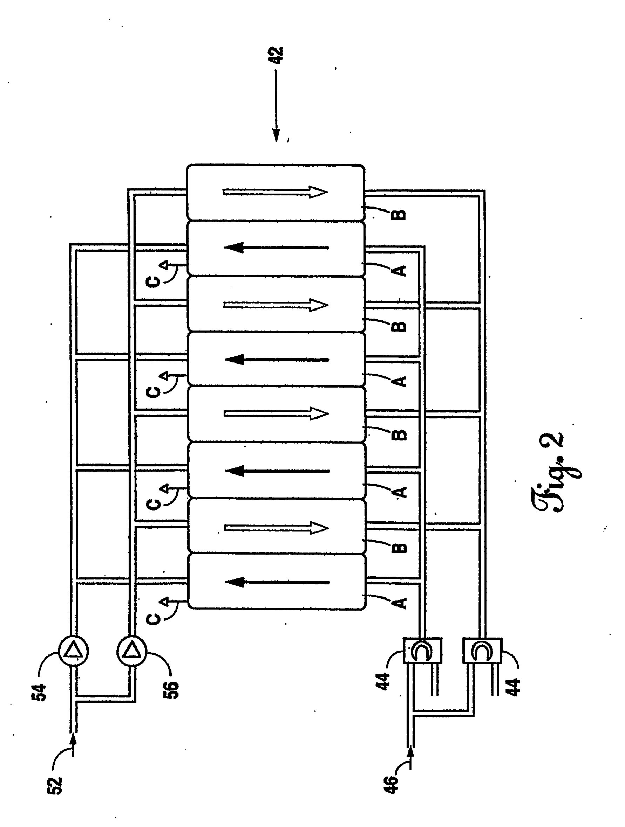

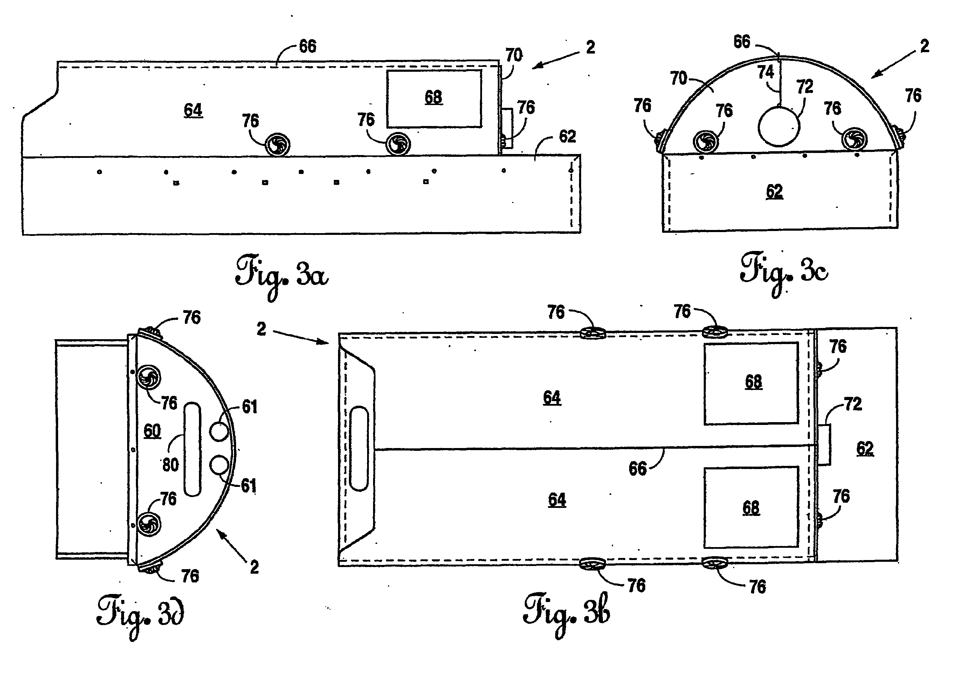

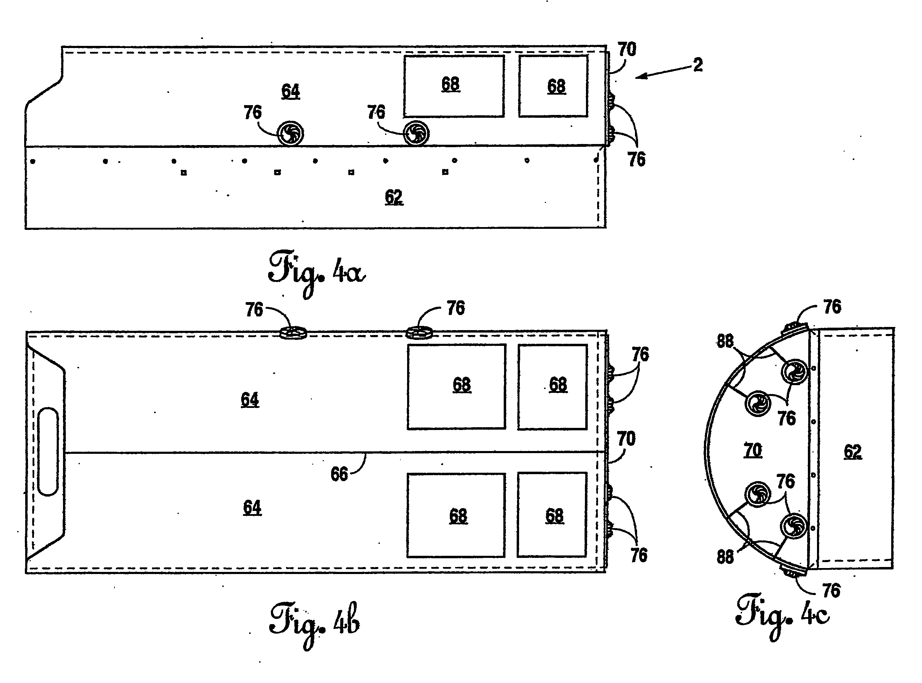

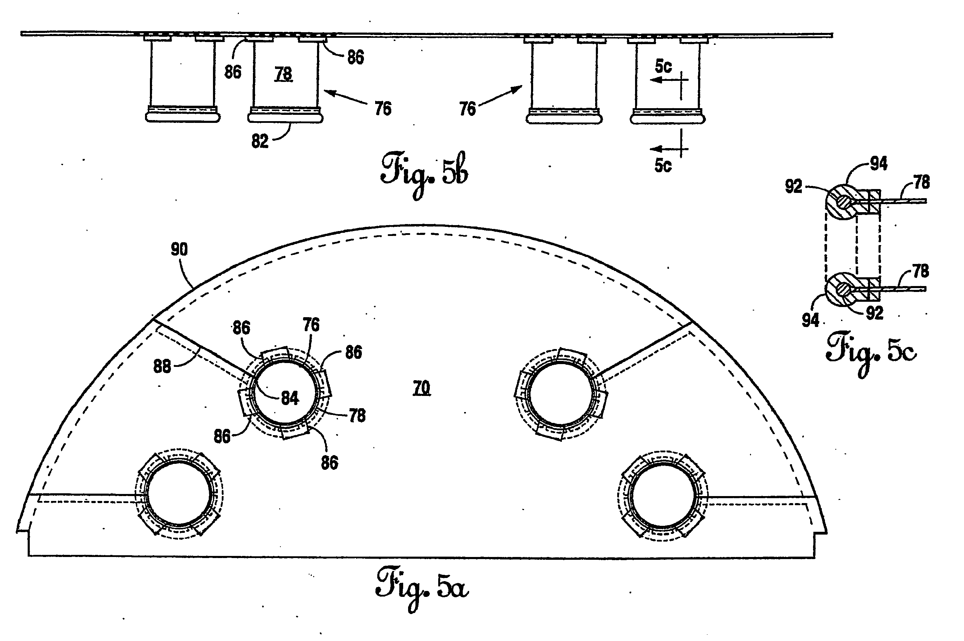

The figure shows, in partially cut away perspective view, the preferred embodiment of the present invention as applied to a mammalian wound site.

### BEST MODE FOR CARRYING OUT THE INVENTION:

Although those of ordinary skill in the art will readily recognize many alternative embodiments, especially in light of the illustrations provided herein, this detailed description is exemplary of the preferred embodiment of the present invention - a vacuum assisted closure system with provision for introduction of agent, the scope of which is limited only by the claims appended hereto.

Referring now to the figure, the present invention 10 is shown to generally comprise a foam pad 11 for insertion substantially into a wound site 12 and a wound drape 13 for sealing enclosure of the foam pad 11 at the wound site 12. According to the invention, the foam pad 11 is placed in fluid communication with a vacuum source for promotion of fluid drainage. Additionally, the foam pad 11 is predisposed, through grafting or other techniques known to those of ordinary skill in the art, with basic fibroblast growth factor (bFGF), anti-microbials or other factors, also known to those of ordinary skill in the art, for the promotion of increased wound healing.

According to the preferred embodiment of the present invention, the foam pad 11, wound drape 13 and vacuum source are implemented as known in the prior art, each of which is detailed in U.S. patent application Serial No. 08/517,901 filed August 22, 1995. By this reference, the full disclosure of U.S. patent application Serial No. 08/517,901 ("the '901 application"), including the claims and the drawings, is incorporated herein as though now set forth in its entirety. Additionally, such a VAC system is readily commercially available through Kinetic Concepts, Inc. of San Antonio, Texas, U.S.A. and/or its subsidiary companies.

As detailed in the '901 application, the foam pad 11 preferably comprises a highly reticulated, open-cell polyurethane or polyether foam for good permeability of wound fluids while under suction, but in this application may comprise a conventional sponge cellulose type dressing as necessary for introduction of the desired agent. As also detailed in the '901 application, the foam pad 11 is preferably placed in fluid communication, via a plastic or like material hose 14, with a vacuum source, which preferably comprises a canister safely placed under vacuum through fluid communication, via an interposed hydrophobic membrane filter, with a vacuum pump. Finally, the '901 application also details the wound drape 13, which preferably comprises an elastomeric material at least peripherally covered with a pressure sensitive, acrylic adhesive for sealing application over the wound site 12.

According to the preferred method of the present invention, those components as are described in the '901 application are generally employed as known in the art with the exception that the foam pad 11 of the present invention is modified as necessary for the introduction of a growth factor. Such growth factors as the basic fibroblast growth factor (bFGF) are known to accelerate wound healing due to their potent angiogenesis and granulation tissue formation activities. As has been demonstrated even with difficult to heal wounds, such as infected wounds, burn wounds and diabetic wounds, the resultant activities lead to the rapid reepitherialization and contraction of the wound. The combination of VAC therapy with growth factor introduction, through the modification of the foam pad 11 and predisposition thereof with the bFGF, is therefore thought to be an important contribution to the wound healing arts. Likewise, the present method presents an excellent opportunity for the introduction to the wound site 12 of anti-microbial agents, whether alone or in combination with bFGF or other agents.

While the foregoing description is exemplary of the preferred embodiment of the present invention, those of ordinary skill in the relevant arts will recognize the many variations, alterations, modifications, substitutions and the like as are readily possible, especially in light of this description, the accompanying drawings and the claims drawn hereto. For example, those of ordinary skill in the art will recognize that while the preferred embodiment of the present invention comprises grafting the desired agent onto the foam pad 11 of the VAC system, those of ordinary skill in the art, with the benefit of this exemplary disclosure, will readily recognize many substantially equivalent modes for introduction of the desired agent. For example, in the case of a foam pad 11 that has not been predisposed with an agent or that has been predisposed with an agent which, over time, has subsequently been exhausted into the wound site 12, the desired agent may be injected with a needle and syringe, or the like, through the wound drape 13 and into the foam pad 11. In any case, because the scope of the present invention is much broader than any particular embodiment, the foregoing detailed description should not be construed as a limitation of the present invention, which is limited only by the claims appended hereto.

### INDUSTRIAL APPLICABILTTY:

The present invention is applicable to the wound healing arts.

## Claims

1. An apparatus for cooling a patient to a temperature below normal body temperature, the apparatus comprising a patient-enclosing air tent having an inlet and outlet connected to an air-cooling system, the air-cooling system recirculating air through the air tent to conserve energy, a plurality of air-bags collectively forming a patient supporting surface, a high air pressure source in fluid communication with the air-bags, and a fluid connection between the air-bags and the air cooling system wherein the air-bags can be pressurised with either relatively low pressure cold air which assists in cooling the patient but provides relatively little support or with relatively high pressure air which is sufficient to support the patient but which provides relatively less cooling effect.

2. The apparatus of claim 1 wherein the plurality of air-bags comprise at least two sets of interleaved air-bags, each set of interleaved air-bags being independently inflatable, wherein each set is operable to be alternatively supplied with relatively low pressure cold air or relatively warm high pressure air while another set is supplied with relatively warm high pressure, or relatively low pressure cold air, in successive alternate cycles of operation, in order to both cool the patient and periodically relieve alternate regions of the patient's body from pressure.
